(19) Europäisches Patentamt
European Patent Office
Office européen des brevets

(11) EP 3 191 076 B1

(12) **EUROPÄISCHE PATENTSCHRIFT**

(45) Veröffentlichungstag und Bekanntmachung des Hinweises auf die Patenterteilung:
**02.10.2019 Patentblatt 2019/40**

(21) Anmeldenummer: **15750754.2**

(22) Anmeldetag: **18.08.2015**

(51) Int Cl.:
***A61K 8/39*** *(2006.01)* ***A61Q 5/06*** *(2006.01)*
***A61Q 5/10*** *(2006.01)* ***A61K 8/86*** *(2006.01)*
***A61K 8/891*** *(2006.01)*

(86) Internationale Anmeldenummer:
**PCT/EP2015/068878**

(87) Internationale Veröffentlichungsnummer:
**WO 2016/037805 (17.03.2016 Gazette 2016/11)**

(54) **FÄRBEMITTEL MIT SPEZIELLEN NICHTIONISCHEN LINEAREN SILICONPOLYMEREN**

DYE COMPOSITION WITH SPECIAL NON-IONIC LINEAR SILICONE POLYMERS

COMPOSITION COLORANTE À BASE DE POLYMÈRES DE SILICONE LINÉAIRE NON IONIQUE PARTICULIERS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.09.2014 DE 102014217994**

(43) Veröffentlichungstag der Anmeldung:
**19.07.2017 Patentblatt 2017/29**

(73) Patentinhaber: **Henkel AG & Co. KGaA**
**40589 Düsseldorf (DE)**

(72) Erfinder:
- **KERL, Sylvia**
  **22763 Hamburg (DE)**
- **BIETZ, Susanne**
  **25336 Elmshorn (DE)**
- **GROSJACQUES, Camille**
  **20255 Hamburg (DE)**

(56) Entgegenhaltungen:
**JP-A- 2005 139 133** **JP-A- 2006 028 119**
**JP-A- 2008 285 415** **US-A1- 2001 042 276**
**US-A1- 2006 123 564** **US-A1- 2009 053 159**
**US-A1- 2010 037 404** **US-A1- 2011 229 425**
**US-A1- 2014 165 301**

- **"Wacker Siliconöle AK", , 1. Januar 2001 (2001-01-01), XP055199592, Gefunden im Internet: URL:https://web.archive.org/web/20091122141733/http://www.drawin.de/dasat/images/2/100212-siliconoleak.pdf [gefunden am 2015-07-01]**
- **"Beauty with Impact", Dow Corning , 2014, XP002745018, Gefunden im Internet: URL:http://www.dowcorning.com/content/publishedlit/27-1304_Hair_Skin_Care_Selection_Guide.pdf [gefunden am 2015-09-24]**
- **DATABASE GNPD [Online] MINTEL; März 2003 (2003-03), "3 Minutes Hair color (pure orange)", XP002745019, Database accession no. 196929**
- **DATABASE GNPD [Online] MINTEL; August 2011 (2011-08), "Step Color", XP002745020, Database accession no. 1613828**
- **"Wacker Silicon?le AK", , 1 January 2001 (2001-01-01), XP055199592, Retrieved from the Internet: URL:https://web.archive.org/web/20091122141733/http://www.drawin.de/dasat/images/2/100212-siliconoleak.pdf [retrieved on 2015-07-01]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 191 076 B1

## Beschreibung

Die vorliegende Erfindung betrifft kosmetische Mittel zur Färbung keratinischer Fasern, welche spezielle nichtionische lineare Siliconpolymere enthalten.

Weiterhin betrifft die vorliegende Erfindung eine Verpackungseinheit (Kit-of-parts), enthaltend ein erfindungsgemäßes kosmetisches Mittel sowie eine Oxidationsmittelzubereitung.

Darüber hinaus betrifft die vorliegende Erfindung ein Verfahren zum Färben von keratinischen Fasern unter Verwendung eines erfindungsgemäßen kosmetischen Mittels sowie einer Oxidationsmittelzubereitung.

Zudem betrifft die vorliegende Erfindung die Verwendung eines erfindungsgemäßen kosmetischen Mittels zur Erhöhung der Buntheit und/oder der Farbtiefe von Farbnuancen.

Schließlich betrifft die vorliegende Erfindung die Verwendung einer erfindungsgemäßen Verpackungseinheit zur Herstellung eines kosmetischen Mittels zur Farbveränderung keratinischer Fasern mit erhöhter Buntheit und/oder Farbtiefe von Farbnuancen.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise mit farbverändernden Mitteln. Diese Mittel sollen neben einer hohen Färbeleistung zusätzliche Eigenschaften, wie beispielsweise die Steigerung des Haarvolumens, aufweisen.

Zur Bereitstellung farbverändernder kosmetischer Mittel, insbesondere für die Haut oder für keratinhaltige Fasern, wie beispielsweise menschliche Haare, sind im Stand der Technik verschiedene Färbesysteme bekannt.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss jedoch üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, welche als färbende Komponente sogenannte Direktzieher enthalten. Hierbei handelt es sich um Farbstoffmoleküle, welche direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als oxidativen Färbungen, so dass sehr viel früher eine vielfach unerwünschte Nuancenverschiebung oder ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Insbesondere bei mehrfacher Anwendung von Mitteln mit 5,6-Dihydroxyindolin ist es möglich, bei Personen mit ergrauten Haaren die natürliche Haarfarbe wiederherzustellen. Die Ausfärbung kann dabei mit Luftsauerstoff als einzigem Oxidationsmittel erfolgen, so dass auf weitere Oxidationsmittel verzichtet werden kann. Bei Personen mit ursprünglich mittelblondem bis braunem Haar kann das 5,6-Dihydroxyindolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Die US 2006/123564 A1 offenbart kosmetische Mittel zur Farbveränderung keratinischer Fasern, enthaltend Oxidationsfarbstoffvorprodukte, nichtionische lineare Polydimethylsiloxane und ethoxylierten primären Alkohol. Diese Zusammensetzungen werden mit einer Oxidationsmittelzubereitung gemischt, die Wasserstoffperoxid und Phosphorsäure enthält.

In der US 2010/037404 A1 werden kosmetische Mittel zur Farbveränderung keratinischer Fasern offenbart, die Oxidationsfarbstoffvorprodukte und Silikone enthalten.

Die im Stand der Technik bekannten oxidativen Färbemittel führen jedoch nicht immer zu der gewünschten hohen Färbeleistung, insbesondere zu einer hohen Buntheit und/oder einer hohen Farbtiefe.

Der vorliegenden Erfindung lag daher die Aufgabe zugrunde, ein kosmetisches Mittel zur Färbung keratinischer Fasern bereitzustellen, welches die Nachteile des Standes der Technik vermeidet bzw. zumindest abschwächt und welches in einer verbesserten Buntheit und/oder Farbtiefe resultiert.

Es wurde nun überraschenderweise gefunden, dass der Zusatz von mindestens einem speziellen nichtionischen linearen Siliconpolymer in kosmetischen Mitteln zur Färbung keratinischer Fasern in einer verbesserten Buntheit von

Farbnuancen resultiert. Ein Indikator für die Buntheit von Farbnuancen ist das Chroma. Je höher der Chromawert ist, desto höher ist die Buntheit der Nuance. Die Buntheit kann optisch noch gesteigert werden, wenn sich die Farbtiefe ebenfalls erhöht. Durch den Zusatz mindestens eines speziellen nichtionischen linearen Siliconpolymers werden erfindungsgemäß ein höherer Chromawert sowie eine erhöhte Farbtiefe erhalten.

Ein erster Gegenstand der Erfindung ist daher ein kosmetisches Mittel zur Farbveränderung keratinischer Fasern, umfassend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,
b) mindestens ein nichtionisches lineares Siliconpolymer der Formel (I)

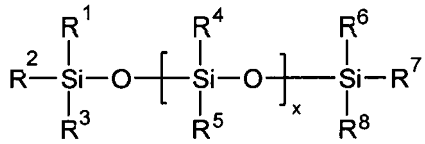
(I),

worin

x für ganze Zahlen von 10 bis 10.000 steht, und
$R^1$ bis $R^7$, jeweils unabhängig voneinander, für Wasserstoff, eine Hydroxygruppe, eine lineare oder verzweigte $C_{1-4}$-Alkylgruppe oder eine lineare oder verzweigte $C_{1-20}$-Hydroxyalkylgruppe stehen,

wobei das kosmetische Mittel mindestens zwei voneinander verschiedene lineare nichtionische Siliconpolymere der Formel I in einer Gesamtmenge von 0,05 bis 1,0 Gew.-%, bezogen auf das Gesamtgewicht des kosmetischen Mittels, in Form einer Emulsion enthält, wobei die Emulsion Teilchen mit einem mittleren Teilchendurchmesser D50 von 0,3 $\mu$m bis 30 $\mu$m aufweist,
a) wobei das erste lineare nichtionische Siliconpolymer (SP1) die Formel (II) sowie ein mittleres Molekulargewicht Mw von 122.000 bis 628.000 Da aufweist

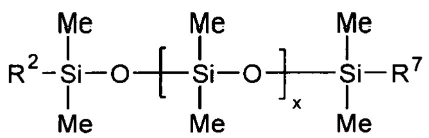
(II),

worin

$R^2$ und $R^7$, jeweils unabhängig voneinander, für eine Methylgruppe stehen, und
x für ganze Zahlen von 1.650 bis 8.500, steht, und

b) wobei das zweite lineare nichtionische Siliconpolymer (SP2) die Formel (III) sowie ein mittleres Molekulargewicht Mw von 3.000 bis 97.000 Da aufweist

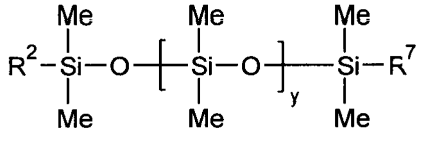
(III),

worin

$R^2$ und $R^7$, jeweils unabhängig voneinander, für eine Methylgruppe stehen, und
y für ganze Zahlen von 40 bis 1.300, steht.

Gemäß obiger Formeln und aller folgenden Formeln steht eine chemische Bindung, welche mit dem Symbol "*" gekennzeichnet ist, für eine freie Valenz des entsprechenden Strukturfragments. Unter freier Valenz ist hierbei die Anzahl von Atombindungen zu verstehen, welche von dem entsprechenden Strukturfragment an der mit dem Symbol

"*" gekennzeichneten Position ausgehen. Im Rahmen der vorliegenden Erfindung geht bevorzugt jeweils eine Atombindung von den mit dem Symbol "*" gekennzeichneten Positionen der Strukturfragmente zu weiteren Strukturfragmenten aus.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn die kosmetischen Mittel zur Färbung von menschlichen Haaren verwendet werden.

Weiterhin werden unter dem Begriff "nichtionische lineare Siliconpolymere" im Rahmen der vorliegenden Erfindung Siliconpolymere verstanden, welche unverzweigt sind und keine permanent anionischen oder permanent kationischen Gruppen sowie keine anionisierbaren oder kationisierbaren Gruppen, wie beispielsweise Carbonsäuregruppen oder Amingruppen, aufweisen.

Zudem wird unter dem Begriff "Kämmbarkeit" im Rahmen der vorliegenden Erfindung sowohl die Kämmbarkeit der nassen Faser, als auch die Kämmbarkeit der trockenen Faser verstanden.

Darüber hinaus werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen erfindungsgemäßen Mittels bezieht sich vorliegend - sofern nichts anderes angegeben ist - auf die Gesamtmenge an Aktivsubstanz der jeweiligen Komponente. Weiterhin bezieht sich die Angabe der Gesamtmenge in Bezug auf die Komponenten des erfindungsgemäßen kosmetischen Mittels - sofern nichts anderes angegeben ist - auf das Gesamtgewicht des oxidationsmittelfreien erfindungsgemäßen kosmetischen Mittels.

Die erfindungsgemäßen kosmetischen Mittel enthalten einen kosmetischen Träger. Erfindungsgemäß bevorzugt ist der kosmetische Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden.

Ein wässriger Träger enthält im Sinne der Erfindung enthält mindestens 30 Gew.-%, insbesondere mindestens 50 Gew.-%, Wasser, bezogen auf das Gesamtgewicht des kosmetischen Mittels.

Unter wässrig-alkoholischen Trägern sind im Sinne der vorliegenden Erfindung wasserhaltige Zusammensetzungen, enthaltend einen $C_1$-$C_4$-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Die erfindungsgemäßen kosmetischen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels, enthalten ist.

Das erfindungsgemäße kosmetische Mittel enthält als ersten wesentlichen Bestandteil a) eine Verbindung, ausgewählt aus der Gruppe von Oxidationsfarbstoffvorprodukten (OFV), direktziehenden Farbstoffe (DZ) sowie deren Mischungen.

In einer bevorzugten Ausführungsform enthalten erfindungsgemäße kosmetische Mittel mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige Verbindungen im erfindungsgemäßen kosmetischen Mittel enthalten sein. In einer bevorzugten Ausführungsform enthalten die erfindungsgemäßen kosmetischen Mittel daher mindestens ein Oxidationsfarbstoffvorprodukt vom Entwicklertyp. Es kann im Rahmen der vorliegenden Erfindung jedoch auch vorgesehen sein, dass die erfindungsgemäßen kosmetischen Mittel mindestens ein Oxidationsfarbstoffvorprodukt vom Kupplertyp enthalten. Besonders gute Ergebnisse in Bezug auf die Färbung keratinischer Fasern werden erhalten, wenn die erfindungsgemäßen kosmetischen Mittel mindestens ein Oxidationsfarbstoffvorprodukt vom Entwicklertyp und mindestens ein Oxidationsfarbstoffvorprodukt vom Kupplertyp enthalten.

Die Entwickler- und Kupplerkomponenten werden üblicherweise in freier Form eingesetzt. Bei Substanzen mit Aminogruppen kann es jedoch bevorzugt sein, deren Salzform, insbesondere in Form der Hydrochloride und Hydrobromide oder der Sulfate, einzusetzen.

Erfindungsgemäß sind kosmetische Mittel bevorzugt, welche die Entwickler- und/oder Kupplerkomponenten jeweils in einer Gesamtmenge von 0,001 bis 10 Gew.-%, vorzugsweise von 0,01 bis 8 Gew.-%, bevorzugt von 0,1 bis

5 Gew.-%, insbesondere von 0,5 bis 3 Gew.-%, bezogen auf das Gesamtgewicht des kosmetischen Mittels, enthalten.

In einer weiteren bevorzugten Ausführungsform ist das erfindungsgemäße kosmetische Mittel daher dadurch gekennzeichnet, dass es ein Oxidationsfarbstoffvorprodukt vom Entwickler und/oder Kupplertyp in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels, enthält.

Geeignete Oxidationsfarbstoffvorprodukte vom Entwicklertyp sind beispielsweise p-Phenylendiamin und dessen Derivate. Bevorzugte p-Phenylendiamine werden ausgewählt aus einer oder mehrerer Verbindungen der Gruppe, welche gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, N,N-Bis-(2-hydroxyethyl)-p-phenylen-diamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methyl-phenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, welche mindestens zwei aromatische Kerne enthalten, die mit Amino- und/oder Hydroxylgruppen substituiert sind. Bevorzugte zweikernige Entwicklerkomponenten werden ausgewählt aus N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan, Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethyl-phenol, 4-Amino-2-(1,2-dihydroxyethyl)phenol, 4-Amino-2-(diethylaminomethyl)phenol sowie deren physiologisch verträglichen Salze.

Ferner kann die Entwicklerkomponente ausgewählt sein aus o-Aminophenol und dessen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind 2,4,5,6-Tetraaminopyrimidin, 4-Hydroxy-2,5,6-triaminopyrimidin und deren physiologisch verträgliche Salze. Ein bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Bevorzugte Oxidationsfarbstoffvorprodukte vom Entwicklertyp sind ausgewählt aus der Gruppe, welche gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen.

Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)-propyl]amin, und/oder 4,5-Diamino-1-(2-hydroxyethyl)pyrazol sowie deren physiologisch verträglichen Salze.

Gemäß einer weiteren bevorzugten Ausführungsform der vorliegenden Erfindung enthält das erfindungsgemäße kosmetische Mittel als Oxidationsfarbstoffvorprodukt neben mindestens einer Entwicklerkomponente weiterhin zusätzlich mindestens eine Kupplerkomponente. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind ausgewählt aus

(A) m-Aminophenol und dessen Derivaten, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,

(B) o-Aminophenol und dessen Derivaten, wie 2-Amino-5-ethylphenol,

(C) m-Diaminobenzol und dessen Derivaten, wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}-amino)ethanol,

(D) o-Diaminobenzol und dessen Derivaten,

(E) Di- beziehungsweise Trihydroxybenzolderivaten, insbesondere Resorcin, 2-Chlorresorcin, 4-Chlorresorcin, 2-Methylresorcin und 1,2,4-Trihydroxybenzol,

(F) Pyridinderivaten, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxy-pyridin,
(G) Naphthalinderivaten, wie 1-Naphthol und 2-Methyl-1-naphthol,
(H) Morpholinderivaten, wie 6-Hydroxybenzomorpholin,
(I) Chinoxalinderivaten,
(J) Pyrazolderivaten, wie 1-Phenyl-3-methylpyrazol-5-on,
(K) Indolderivaten, wie 6-Hydroxyindol,
(L) Pyrimidinderivaten oder
(M) Methylendioxybenzolderivaten, wie 1-(2'-Hydroxyethyl)-amino-3,4-methylendioxybenzol sowie deren physiologisch verträglichen Salze.

Erfindungsgemäß bevorzugte Kupplerkomponenten sind ausgewählt aus der Gruppe, welche gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-di-aminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie deren physiologisch verträgliche Salze.

In einer besonders bevorzugten Ausführungsform der vorliegenden Erfindung sind die erfindungsgemäßen kosmetischen Mittel dadurch gekennzeichnet, dass sie als Oxidationsfarbstoffvorprodukt mindestens eine Entwicklerkomponente, ausgewählt aus der Gruppe von p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol, deren physiologisch verträglichen Salze sowie deren Mischungen, und mindestens eine Kupplerkomponente, ausgewählt aus der Gruppe von Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin, deren physiologisch verträglichen Salze sowie deren Mischungen, enthalten.

Um eine ausgewogene und subtile Nuancenausbildung zu erhalten, kann es im Rahmen der vorliegenden Erfindung auch vorgesehen sein, dass die erfindungsgemäßen kosmetischen Mittel zusätzlich mindestens einen direktziehenden Farbstoff enthalten. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, welche mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC

Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Bevorzugt enthält das erfindungsgemäße kosmetische Mittel die direktziehenden Farbstoffe in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels.

Als zweiten wesentlichen Bestandteil b) enthalten die erfindungsgemäßen kosmetischen Mittel mindestens zwei voneinander verschiedene lineare nichtionische Siliconpolymere in einer Gesamtmenge von 0,05 bis 1,0 Gew.-%, bezogen auf das Gesamtgewicht des kosmetischen Mittels. Der Zusatz dieser speziellen Siliconpolymere führt zu einer verbesserten Buntheit und/oder Farbtiefe von Farbnuancen, so dass eine geringere Menge an Farbstoffen bzw. Farbstoffvorprodukten, wie Entwicklerkomponenten, Kupplerkomponenten, direktziehenden Farbstoffen sowie deren Mischungen, eingesetzt werden muss, um eine identische Buntheit zu erhalten wie bei Verwendung von kosmetischen Mitteln ohne spezielle nichtionische lineare Siliconpolymere.

Gemäß einer bevorzugten Ausführungsform der vorliegenden Erfindung stehen in der Struktureinheit der Formel (I) die Reste $R^1$ bis $R^7$, jeweils unabhängig voneinander, für eine lineare $C_{1-4}$-Alkylgruppe, insbesondere für eine Methylgruppe.

Besonders gute Ergebnisse im Rahmen der vorliegenden Erfindung werden erhalten, wenn in der Struktureinheit der Formel (I) die Reste $R^1$ bis $R^7$, jeweils unabhängig voneinander, für eine lineare $C_{1-4}$-Alkylgruppe, insbesondere für eine Methylgruppe stehen und x für ganze Zahlen von 1.500 bis 10.000, vorzugsweise von 1.550 bis 9.500, bevorzugt von 1.600 bis 9.000, insbesondere von 1.650 bis 8.500, steht.

Im Rahmen dieser Ausführungsform ist es bevorzugt, wenn das nichtionische lineare Siliconpolymer der Formel (I) ein mittleres Molekulargewicht $M_w$ von 111.100 bis 740.000 Da, vorzugsweise von 114.000 bis 703.000 Da, bevorzugt von 118.000 bis 665.000 Da, insbesondere von 122.000 bis 628.000 Da, aufweist. Das mittlere Molekulargewicht $M_w$ kann beispielsweise durch Gelpermeationschromatographie (GPC) bestimmt werden (Liu X. M. et. al.; "Comparative Studies of Poly(DimethylSiloxanes) Using Automated GPC-MALDI-TOF MS and On-Line GPC-ESI-TOF MS"; Am. Soc. Mass. Spectrom., 2003, 14, Seiten 195 bis 202).

Weiterhin werden besonders gute Ergebnisse erhalten, wenn in der Struktureinheit der Formel (I) die Reste $R^1$ bis $R^7$, jeweils unabhängig voneinander, für eine lineare $C_{1-4}$-Alkylgruppe, insbesondere für eine Methylgruppe stehen und x für ganze Zahlen von 1.500 bis 10.000, vorzugsweise von 1.550 bis 9.500, bevorzugt von 1.600 bis 9.000, insbesondere von 1.650 bis 8.500, steht.

Im Rahmen dieser Ausführungsform ist es erfindungsgemäß bevorzugt, wenn das nichtionische lineare Siliconpolymer der Formel (I) ein mittleres Molekulargewicht $M_w$ von 111.100 bis 740.000 Da, vorzugsweise von 114.000 bis 703.000 Da, bevorzugt von 118.000 bis 665.000 Da, insbesondere von 122.000 bis 628.000 Da, aufweist. Die Bestimmung des mittleren Molekulargewichts $M_w$ des speziellen nichtionischen Siliconpolymers kann bevorzugt durch die zuvor genannte GPC-Methode erfolgen. Erfindungsgemäß enthält das das kosmetische Mittel mindestens zwei voneinander verschiedene lineare nichtionische Siliconpolymere,

a) wobei das erste lineare nichtionische Siliconpolymer (SP1) die Formel (II) aufweist

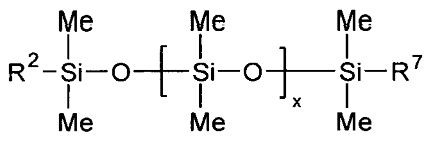

(II),

worin

$R^2$ und $R^7$, jeweils unabhängig voneinander, für eine Methylgruppe stehen, und
x für ganze Zahlen von 1.650 bis 8.500, steht, und

b) wobei das zweite lineare nichtionische Siliconpolymer (SP2) die Formel (III) aufweist

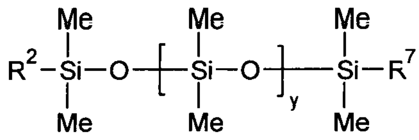
(III),

worin

$R^2$ und $R^7$, jeweils unabhängig voneinander, für eine Methylgruppe stehen, und

y für ganze Zahlen von 40 bis 1.300, steht.

Der Einsatz dieser Mischung aus zwei speziellen nichtionischen linearen Siliconpolymeren (SP1) und (SP2) mit unterschiedlichen mittleren Molekulargewichten $M_w$ resultiert in einer verbesserten Buntheit und/oder Farbtiefe von Farbnuancen. Hierdurch kann die Menge an eingesetztem Farbstoff im Vergleich zu kosmetischen Mitteln, welche keine speziellen nichtionischen linearen Siliconpolymere enthalten, deutlich reduziert werden, ohne jedoch die Buntheit der entsprechenden Farbnuancen negativ zu beeinflussen.

Erfindungsgemäß weist damit das nichtionische lineare Siliconpolymer (SP1) der Formel (II) ein mittleres Molekulargewicht $M_w$ von 122.000 bis 628.000 Da, auf und das nichtionische lineare Siliconpolymer (SP2) der Formel (III) ein mittleres Molekulargewicht $M_w$ von 3.000 bis 97.000 Da, auf. Bei Einsatz der Mischung von nichtionischen linearen Siliconpolymeren (SP1) und (SP2) mit den zuvor angeführten unterschiedlichen mittleren Molekulargewichten $M_w$ werden besonders gute Ergebnisse im Hinblick auf die erhöhte Buntheit und/oder Farbtiefe von Farbnuancen erhalten.

Erfindungsgemäß liegen die mindestens zwei nichtionischen linearen Siliconpolymere b) in Form einer Emulsion vor, wobei die Emulsion Teilchen mit einem mittleren Teilchendurchmesser $D_{50}$ von 0,3 $\mu$m bis 30 $\mu$m aufweist. Spezielle nichtionische lineare Siliconpolymere, welche als Emulsion mit der zuvor genannten Teilchengröße vorliegen, resultieren in einer besonders hohen Buntheit und/oder Farbtiefe von Farbnuancen. Die Bestimmung des mittleren Teilchendurchmessers Dso ist beispielsweise mittels dynamischer Lichtstreuung (DLS) möglich.

Das erfindungsgemäße kosmetische Mittel enthält mindestens zwei voneinander verschiedene lineare nichtionische Siliconpolymere in einer Gesamtmenge von 0,05 bis 1,0 Gew.-%, bezogen auf das Gesamtgewicht des kosmetischen Mittels. Der Einsatz der zuvor genannten Gesamtmenge der mindestens zwei speziellen nichtionischen linearen Siliconpolymere führt zu einer verbesserten Buntheit und/oder Farbtiefe von Farbnuancen. Weiterhin stellen die zuvor genannten Mengen der speziellen nichtionischen linearen Siliconpolymere sicher, dass keine Inkompatibilitäten mit weiteren Inhaltsstoffen des erfindungsgemäßen kosmetischen Mittels auftreten, so dass bei Verwendung dieser Mengen eine hohe Lagerstabilität der kosmetischen Mittel gewährleistet wird.

Es hat sich gezeigt, dass ein Zusatz an Polyoxyethylen(23)-laurylether die mindestens zwei nichtionischen linearen Siliconpolymere, insbesondere die nichtionischen linearen Siliconpolymere der Formeln (II) und (III), in den erfindungsgemäßen kosmetischen Mitteln stabilisieren kann, so dass die Buntheit und/oder Farbtiefe von Farbnuancen weitergehend verstärkt werden kann. Erfindungsgemäß bevorzugte kosmetische Mittel enthalten daher zusätzlich Polyoxyethylen-(23)-laurylether in einer Gesamtmenge von $1x10^{-5}$ bis 1,5 Gew.-%, vorzugsweise von $3x10^{-5}$ bis 0,8 Gew.-%, bevorzugt von $1x10^{-4}$ bis 0,4 Gew.-%, weiter bevorzugt von $3x10^{-4}$ bis 0,2 Gew.-%, insbesondere von $1x10^{-3}$ bis 0,05 Gew.-%, bezogen auf das Gesamtgewicht des kosmetischen Mittels, bezogen auf das Gesamtgewicht des kosmetischen Mittels.

Die mindestens zwei nichtionischen linearen Siliconpolymere, insbesondere die nichtionischen linearen Siliconpolymere der Formeln (II) und (III), kann weiterhin durch den Zusatz eines ethoxylierten primären Alkohols stabilisiert werden. Es ist daher im Rahmen der vorliegenden Erfindung bevorzugt, wenn die erfindungsgemäßen kosmetischen Mittel zusätzlich einen ethoxylierten primären Alkohol in einer Gesamtmenge von $5x10^{-6}$ bis 1,0 Gew.-%, vorzugsweise von $1x10^{-5}$ bis 0,4 Gew.-%, bevorzugt von $5x10^{-5}$ bis 0,3 Gew.-%, weiter bevorzugt von $1x10^{-4}$ bis 0,2 Gew.-%, insbesondere von $5x10^{-4}$ bis 0,01 Gew.-%, bezogen auf das Gesamtgewicht des kosmetischen Mittels, enthalten, wobei der ethoxylierte primäre Alkohol die Formel (IV) aufweist

$$C_nH_{2n+1}(CH_2CH_2O)_m\text{-}CH_2\text{-}CH_2\text{-}OH \qquad \text{(IV)},$$

worin n für ganze Zahlen von 8 bis 20, vorzugsweise von 10 bis 18, insbesondere von 12 bis 15, steht und m für ganze Zahlen von 1 bis 8, vorzugsweise von 1 bis 6, bevorzugt von 2 bis 5, insbesondere für 2 oder 3, steht. Als ethoxylierter primärer Alkohol eignet sich im Rahmen der vorliegenden Erfindung beispielsweise die unter INCI-Bezeichnung $C_{12\text{-}15}$ Pareth-3 bekannte Verbindung.

Die erfindungsgemäßen kosmetischen Mittel können weitere Wirk- und Zusatzstoffe enthalten. Es ist daher im Rahmen der vorliegenden Erfindung bevorzugt, wenn das kosmetische Mittel zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

Bevorzugt werden die erfindungsgemäßen kosmetischen Mittel als fließfähige Zubereitungen formuliert. Dabei sollten die kosmetischen Mittel so formuliert werden, dass diese sich einerseits gut am Anwendungsort auftragen und verteilen lassen, andererseits jedoch ausreichend viskos sind, so dass sie während der Einwirkzeit am Wirkort verbleiben und nicht verlaufen.

Es hat sich daher erfindungsgemäß als vorteilhaft erwiesen, wenn die erfindungsgemäßen kosmetischen Mittel mindestens ein Verdickungsmittel aus der Gruppe von (i) anionischen, synthetischen Polymeren; (ii) kationischen, synthetischen Polymeren; (iii) natürlich vorkommenden Verdickungsmitteln, wie nichtionischen Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektinen, Alginaten, Stärke-Fraktionen und Derivaten, wie Amylose, Amylopektin und Dextrinen, sowie Cellulosederivaten, wie beispielsweise Methylcellulose, Carboxyalkylcellulosen und Hydroxyalkylcellulosen; (iv) nichtionischen, synthetischen Polymeren, wie Polyvinylalkohol oder Polyvinylpyrrolidinon; (v) anorganischen Verdickungsmitteln, insbesondere Schichtsilikaten wie beispielsweise Bentonit, besonders Smektite, wie Montmorillonit oder Hectorit; sowie (vi) deren Mischungen, in einer Gesamtmenge von 0,0005 bis 5,0 Gew.-%, vorzugsweise von 0,001 bis 3,0 Gew.-%, bevorzugt von 0,005 bis 1,0 Gew.-%, insbesondere von 0,008 bis 0,01 Gew.-%, bezogen auf das Gesamtgewicht des kosmetischen Mittels, enthalten.

Es hat sich im Rahmen der vorliegenden Erfindung als vorteilhaft erwiesen, wenn als Verdickungsmittel mindestens ein natürlich vorkommendes Verdickungsmittel, insbesondere Xanthangum sowie dessen Salze, in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,1 Gew.-%, bezogen auf das Gesamtgewicht des kosmetischen Mittels, enthalten ist.

Im Rahmen der vorliegenden Erfindung kann es bevorzugt sein, wenn der lineare oder verzweigte, gesättigte oder ungesättigte Alkohol mit 8 bis 20 Kohlenstoffatomen ausgewählt ist aus der Gruppe von Myristylalkohol (1-Tetradecanol), Stearylalkohol (1-Octadecanol), Cetearylalkohol, 2-Octyldodecanol, Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), vorzugsweise 2-Octyldodecanol und/oder Cetearylalkohol, und in einer Gesamtmenge von 1,0 bis 35 Gew.-%, vorzugsweise von 5,0 bis 30 Gew.-%, bevorzugt von 10 bis 25 Gew.-%, insbesondere von 12 bis 20 Gew.-%, bezogen auf das Gesamtgewicht des kosmetischen Mittels, enthalten ist.

Bevorzugt können die erfindungsgemäßen kosmetischen Mittel weiterhin mindestens einen Partialester aus einem Polyol mit 2 bis 6 Kohlenstoffatomen und linearen gesättigten Carbonsäuren mit 12 bis 30, insbesondere 14 bis 22 Kohlenstoffatomen, wobei die Partialester hydroxyliert sein können, in einer Gesamtmenge von 0,5 bis 10 Gew.-%, insbesondere von 3,0 bis 8,0 Gew.-%, bezogen auf das Gesamtgewicht des kosmetischen Mittels, enthalten. Solche Partialester sind insbesondere die Mono- und Diester von Glycerin oder die Monoester von Propylenglycol oder die Mono- und Diester von Ethylenglycol oder die Mono-, Di-, Tri- und Tetraester von Pentaerythrit jeweils mit linearen gesättigten $C_{12}$ - $C_{30}$-Carbonsäuren, welche hydroxyliert sein können, insbesondere diejenigen mit Palmitin- und Stearinsäure, die Sorbitanmono-, -di- oder -triester von linearen gesättigten $C_{12}$ - $C_{30}$-Carbonsäuren, welche hydroxyliert sein können, insbesondere diejenigen von Myristinsäure, Palmitinsäure, Stearinsäure oder von Mischungen dieser Fettsäuren und die Methylglucosemono- und -diester von linearen gesättigten $C_{12}$ - $C_{30}$-Carbonsäuren, welche hydroxyliert sein können.

Im Rahmen der vorliegenden Erfindung kann es vorgesehen sein, dass die erfindungsgemäßen kosmetischen Mittel mindestens einen Polyolpartialester, ausgewählt aus Glycerinmonostearat, Glycerinmonopalmitat, Glycerindistearat, Glycerindipalmitat, Ethylenglycolmonostearat, Ethylenglycolmonopalmitat, Ethylenglycoldistearat, Ethylenglycoldipalmitat, sowie Mischungen hiervon, insbesondere Mischungen aus Glycerinmonostearat, Glycerinmonopalmitat, Glycerindistearat und Glycerindipalmitat in einer Gesamtmenge von 0,5 bis 10 Gew.-%, insbesondere von 3,0 bis 8,0 Gew.-%, bezogen auf das Gesamtgewicht des kosmetischen Mittels, enthalten.

Der Einsatz der zuvor angeführten Alkohole, Partialester und Polypartialester in den erfindungsgemäßen kosmetischen Mitteln kann insbesondere dann bevorzugt sein, wenn die erfindungsgemäßen kosmetischen Mittel in Form einer Öl-in-Wasser-Emulsion vorliegen.

Weiterhin kann es erfindungsgemäß vorgesehen sein, dass die kosmetischen Mittel gemäß der Erfindung mindestens ein Tensid enthalten. Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Gemäß einer bevorzugten Ausführungsform der vorliegenden Erfindung enthalten die erfindungsgemäßen kosmetischen Mittel mindestens ein amphoteres Tensid in einer Gesamtmenge von 0,1 bis 5,0 Gew.-%, insbesondere von 0,2 bis 2,0 Gew.-%, bezogen auf das Gesamtgewicht des kosmetischen Mittels. Als amphotere bzw. zwitterionische Tenside werden solche oberflächenaktiven Verbindungen bezeichnet, welche mindestens eine quartäre Ammoniumgruppe und mindestens eine - $COO^{(-)}$- oder -$SO3^{(-)}$-Gruppe aufweisen.

Als amphotere Tenside sind im Rahmen der vorliegenden Erfindung die nachfolgend genannten Verbindungen besonders bevorzugt:

- Alkylbetaine mit 8 bis 20 Kohlenstoffatomen in der Alkylgruppe,
- Amidopropylbetaine mit 8 bis 20 Kohlenstoffatomen in der Acylgruppe,
- Sulfobetaine mit 8 bis 20 Kohlenstoffatomen in der Acylgruppe, und
- Amphoacetate oder Amphodiacetate mit 8 bis 20 Kohlenstoffatomen in der Acylgruppe.

In einer besonders bevorzugten Ausführungsform enthalten die erfindungsgemäßen kosmetischen Mittel als Tensid mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, bezogen auf das Gesamtgewicht des kosmetischen Mittels.

Weiterhin kann es vorgesehen sein, dass die erfindungsgemäßen kosmetischen Mittel mindestens ein ethoxyliertes nichtionisches Tensid in einer Gesamtmenge von 0,5 bis 6,0 Gew.-%, insbesondere von 1,0 bis 4,0 Gew.-%, bezogen auf das Gesamtgewicht des kosmetischen Mittels, enthalten. Die ethoxylierten nichtionischen Tenside sind hierbei von den zuvor angeführten ethoxylierten primären Alkoholen verschieden. Dabei hat es sich als besonders vorteilhaft erwiesen, wenn das ethoxylierte nichtionische Tensid einen HLB-Wert oberhalb von 10, vorzugsweise oberhalb von 13 aufweist. Hierzu ist es erforderlich, dass das nichtionische Tensid einen ausreichend hohen Ethoxylierungsgrad besitzt. In diesem Zusammenhang enthält das erfindungsgemäße kosmetische Mittel daher als ethoxyliertes nichtionisches Tensid mindestens ein ethoxyliertes Tensid mit mindestens 12 Ethylenoxideinheiten. Neben den entsprechend ethoxylierten Fettalkoholen, insbesondere Laurylalkohol, Myristylalkohol, Cetylalkohol, Stearylalkohol, Arachylalkohol und Behenylalkohol, sind erfindungsgemäß insbesondere die Anlagerungsprodukte von 20 bis 60 Mol Ethylenoxid an Rizinusöl und gehärtetes Rizinusöl besonders geeignet. Das mindestens eine ethoxylierte nichtionische Tensid ist bevorzugt ausgewählt aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30.

Kosmetische Mittel im Rahmen der vorliegenden Erfindung weisen in der Regel einen basischen pH-Wert, insbesondere zwischen pH 8,0 und pH 12, auf. Diese pH-Werte sind erforderlich, um eine Öffnung der äußeren Schuppenschicht (Cuticula) zu gewährleisten und eine Penetration der Oxidationsfarbstoffvorprodukte und/oder des Oxidationsmittels in das Haar zu ermöglichen.

Die Einstellung des zuvor genannten pH-Wertes kann bevorzugt unter Verwendung eines Alkalisierungsmittels erfolgen. Im Rahmen der vorliegenden Erfindung ist das Alkalisierungsmittel ausgewählt aus der Gruppe von (i) anorganischen Alkalisierungsmitteln; (ii) organischen Alkalisierungsmitteln; sowie (iii) deren Mischungen, und in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels, enthalten.

Bevorzugte anorganische Alkalisierungsmittel sind ausgewählt aus der Gruppe, welche gebildet wird aus Ammoniak bzw. Ammoniumhydroxid, also wässrigen Lösungen von Ammoniak, Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie Mischungen hiervon. Ammoniak bzw. Ammoniumhydroxid ist ein besonders bevorzugtes Alkalisierungsmittel. Besonders bevorzugt ist Ammoniak in einer Gesamtmenge von 0,1 bis 20 Gew.-%, vorzugsweise von 0,5 bis 10 Gew.-%, insbesondere von 1,0 bis 7,0 Gew.-%, bezogen auf das Gesamtgewicht des kosmetischen Mittels, enthalten.

Bevorzugte organische Alkalisierungsmittel sind ausgewählt aus mindestens einem Alkanolamin. Erfindungsgemäß bevorzugte Alkanolamine sind ausgewählt aus Alkanolaminen aus primären, sekundären oder tertiären Aminen mit einem $C_2$-$C_6$-Alkylgrundkörper, welcher mindestens eine Hydroxylgruppe trägt. Besonders bevorzugte Alkanolamine werden aus der Gruppe ausgewählt, welche gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin. Erfindungsgemäß ganz besonders bevorzugte Alkanolamine werden ausgewählt aus der Gruppe von 2-Aminoethan-1-ol (Monoethanolamin), 2-Amino-2-methyl-propan-1-ol, 2-Amino-2-methyl-propan-1,3-diol und Triethanolamin. Besonders bevorzugte erfindungsgemäße kosmetische Mittel enthalten eine Mischung aus Monoethanolamin und 2-Amino-2-methylpropan-1ol. Bevorzugt ist das mindestens eine Alkanolamin in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels, enthalten.

Weitere erfindungsgemäß bevorzugte organische Alkalisierungsmittel sind ausgewählt aus basischen Aminosäuren, besonders bevorzugt ausgewählt aus der Gruppe, welche gebildet wird aus L-Arginin, D-Arginin, D/L-Arginin, L-Lysin, D-Lysin, D/L-Lysin sowie Mischungen hiervon. Erfindungsgemäß besonders bevorzugte basische Aminosäuren sind ausgewählt aus L-Arginin, D-Arginin und D/L-Arginin. Bevorzugte erfindungsgemäße kosmetische Mittel enthalten mindestens ein von Alkanolaminen und Ammoniak verschiedenes Alkalisierungsmittel in einer Gesamtmenge von 0,05 bis 5,0 Gew.-%, insbesondere von 0,5 bis 3,0 Gew.-%, bezogen auf das Gesamtgewicht des kosmetischen Mittels.

In einer besonders bevorzugten Ausführungsform enthalten die erfindungsgemäßen kosmetischen Mittel als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels.

Bevorzugt beträgt der pH-Wert der erfindungsgemäßen kosmetischen Mittel, gemessen bei 22°C, 8 bis 13, vorzugsweise 9,5 bis 12, bevorzugt 10 bis 11,5, insbesondere 10,5 bis 11.

Im Rahmen der vorliegenden Erfindung kann es weiterhin bevorzugt sein, wenn die erfindungsgemäßen kosmetischen Mittel mindestens ein Öl, ausgewählt aus der Gruppe von Sonnenblumenöl, Maisöl, Sojaöl, Kürbiskernöl, Traubenkernöl, Sesamöl, Haselnussöl, Aprikosenkernöl, Macadamianussöl, Araraöl, Rizinusöl, Avocadoöl sowie deren Mischungen, in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels, enthalten. Durch den Einsatz mindestens eines zuvor genannten Öls kann der Pflegeeffekt der nichtionischen linearen Siliconpolymere weitergehend gesteigert werden.

Besonders bevorzugt enthalten die erfindungsgemäßen kosmetischen Mittel Traubenkernöl in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels.

Gemäß einer besonders bevorzugten Ausführungsform der vorliegenden Erfindung enthalten die als Ölin-Wasser-Emulsion vorliegenden erfindungsgemäßen kosmetischen Mittel - bezogen auf das Gesamtgewicht der kosmetischen Mittel -

- Cetearylalkohol in einer Gesamtmenge von 2,0 bis 20 Gew.-%, insbesondere von 5,0 bis 12 Gew.-%, weiterhin
- Mischungen aus Glycerinmonostearat, Glycerinmonopalmitat, Glycerindistearat und Glycerindipalmitat in einer Gesamtmenge von 0,5 bis 10 Gew.-%, bevorzugt 3,0 bis 8,0 Gew.-%, weiterhin
- mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, weiterhin
- eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, weiterhin
- Traubenkernöl in einer Gesamtmenge von 0,1 bis 10 Gew.-%, bevorzugt von 0,2 bis 5,0 Gew.-%, insbesondere von 0,5 bis 2,0 Gew.-%.

Oxidative Färbezusammensetzungen können auch direkt vor der Anwendung aus zwei oder mehreren getrennt verpackten Zusammensetzungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme ist insbesondere dort bevorzugt, wo Inkompatibilitäten der Inhaltsstoffe zu erwarten oder zu befürchten sind. Die oxidative Färbezusammensetzung wird in diesen Fällen vom Verbraucher direkt vor der Anwendung durch Vermischen der Komponenten hergestellt. Im Rahmen der vorliegenden Erfindung ist dieses Vorgehen bei oxidativen Färbemitteln, bei welchen das erfindungsgemäße kosmetische Mittel zunächst getrennt von einer Oxidationsmittelzubereitung, enthaltend mindestens ein Oxidationsmittel, vorliegt, besonders bevorzugt.

Ein weiterer Gegenstand der vorliegenden Erfindung ist daher eine Verpackungseinheit (Kit-of-Parts), umfassend - getrennt voneinander konfektioniert -

a) mindestens einen Container (C1), enthaltend ein erfindungsgemäßes kosmetisches Mittel, und
b) mindestens einen Container (C2), enthaltend eine Oxidationsmittelzubereitung, welche in einem kosmetisch verträglichen Träger mindestens ein Oxidationsmittel sowie mindestens eine Säure enthält.

Die aus der erfindungsgemäßen Verpackungseinheit herstellbaren oxidativen Färbemittel, welche mindestens zwei spezielle nichtionische lineare Siliconpolymere enthalten, führen zu einer verbesserten Buntheit und/oder Farbtiefe der mit diesen Färbemitteln gefärbten keratinischen Fasern. Um die gleiche Buntheit zu erzielen wie bei der Verwendung herkömmlicher oxidativer Färbemittel ohne spezielle nichtionische linearen Siliconpolymere kann bei den aus der erfindungsgemäßen Verpackungseinheit hergestellten oxidativen Färbemitteln die Menge an Farbstoffen deutlich reduziert werden.

Unter dem Begriff "Container" wird im Rahmen der vorliegenden Erfindung eine Umhüllung verstanden, welche in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich dabei jedoch um Umhüllungen aus Glas oder Kunststoff.

Die Oxidationsmittel im Rahmen der vorliegenden Erfindung sind von Luftsauerstoff verschieden. Als Oxidationsmittel können Wasserstoffperoxid sowie dessen feste Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt. Erfindungsgemäß bevorzugt ist das Oxidationsmittel daher ausgewählt aus der Gruppe von Persulfaten, Chloriten, Wasserstoffperoxid und Anlagerungsprodukten von Wasserstoffperoxid an Harnstoff, Melamin sowie Natriumborat, insbesondere Wasserstoffperoxid.

Ein besonders bevorzugte Ausführungsform der vorliegenden Erfindung ist somit dadurch gekennzeichnet, dass als Oxidationsmittel Wasserstoffperoxid in einer Gesamtmenge von 0,5 bis 15 Gew.-%, vorzugsweise von 0,75 bis 10 Gew.-%, bevorzugt von 1 bis 7,5 Gew.-%, besonders bevorzugt von 1,25 bis 7 Gew.-%, insbesondere von 1,5 bis 6,0 Gew.-%, bezogen auf das Gesamtgewicht der Oxidationsmittelzubereitung, enthalten ist. Die Berechnung der Gesamtmenge bezieht sich hierbei auf 100 %-iges $H_2O_2$.

Die Oxidationsmittelzubereitungen können weiterhin Wasser in einer Gesamtmenge von 40 bis 98 Gew.-%, insbesondere von 65 bis 85 Gew.-%, bezogen auf das Gesamtgewicht der Oxidationsmittelzubereitung, enthalten.

Gemäß einer bevorzugten Ausführungsform der vorliegenden Erfindung enthalten die Oxidationsmittelzubereitungen weiterhin mindestens ein lineares gesättigtes Alkanol mit 12 bis 30 Kohlenstoffatomen, insbesondere mit 16 bis 22 Kohlenstoffatomen, in einer Gesamtmenge von 0,1 bis 10 Gew.-%, vorzugsweise von 0,5 bis 5,0 Gew.-%, insbesondere von 1,0 bis 4,0 Gew.-%, bezogen auf das Gesamtgewicht der Oxidationsmittelzubereitung. Bevorzugt sind insbesondere Cetylalkohol, Stearylalkohol, Arachidylalkohol, Behenylalkohol und Lanolinalkohol oder Gemische dieser Alkohole, wie sie bei der großtechnischen Hydrierung von pflanzlichen und tierischen Fettsäuren erhältlich sind, sowie Mischungen dieser Alkanole. Besonders bevorzugt ist die Mischung Cetearylalkohol.

In einer weiteren bevorzugten Ausführungsform der vorliegenden Erfindung enthalten die Oxidationsmittelzubereitungen mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1 bis 4,0 Gew.-%, bezogen auf das Gesamtgewicht der Oxidationsmittelzubereitung.

Im Rahmen der vorliegenden Erfindung kann es darüber hinaus auch vorgesehen sein, dass die Oxidationsmittelzubereitungen mindestens einen Ester aus einer Carbonsäure mit 10 bis 20 Kohlenstoffatomen und einem linearen oder verzweigten Alkohol mit 1 bis 5 Kohlenstoffatomen, insbesondere Isopropylmyristat, in einer Gesamtmenge von 3,0 bis 25 Gew.-%, vorzugsweise von 5,0 bis 20 Gew.-%, insbesondere von 8,0 bis 15 Gew.-%, bezogen auf das Gesamtgewicht der Oxidationsmittelzubereitung, enthalten.

Gemäß einer besonders bevorzugten Ausführungsform der vorliegenden Erfindung enthalten die Oxidationsmittelzubereitungen - bezogen auf das Gesamtgewicht der Oxidationsmittelzubereitungen -

- mindestens ein lineares gesättigtes Alkanol mit 12 bis 30 Kohlenstoffatomen in einer Gesamtmenge von 0,1 bis 10 Gew.-%, vorzugsweise von 0,5 bis 5,0 Gew.-%, insbesondere von 1,0 bis 4,0 Gew.-%, weiterhin
- mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1,0 bis 4,0 Gew.-%, sowie
- mindestens einen Ester aus einer Carbonsäure mit 10 bis 20 Kohlenstoffatomen und einem linearen oder verzweigten Alkohol mit 1 bis 5 Kohlenstoffatomen, bevorzugt Isopropylmyristat, in einer Gesamtmenge von 3,0 bis 25 Gew.-%, vorzugsweise von 5,0 bis 20 Gew.-%, insbesondere von 8,0 bis 15 Gew.-%.

Die erfindungsgemäßen Oxidationsmittelzubereitungen enthalten weiterhin mindestens eine Säure. Bevorzugte Säuren sind ausgewählt aus Dipicolinsäure, Genusssäuren, wie beispielsweise Zitronensäure, Essigsäure, Apfelsäure, Milchsäure und Weinsäure, verdünnten Mineralsäuren, wie Salzsäure, Phosphorsäure, Pyrophosphorsäure und Schwefelsäure, sowie Mischungen hiervon. Die Oxidationsmittelzubereitungen weisen bevorzugt einen pH-Wert im Bereich von 2 bis 5, insbesondere von 3 bis 4, auf.

Zur Herstellung von oxidativen Färbezusammensetzungen aus der erfindungsgemäßen Verpackungseinheit (Kit-of-parts) wird das erfindungsgemäße kosmetische Mittel in dem Container C1 mit der Oxidationsmittelzubereitung in dem Container C2 oder *vice versa* vermischt.

Weiterhin kann es erfindungsgemäß besonders vorteilhaft sein, wenn die Verpackungseinheit mindestens ein weiteres Haarbehandlungsmittel, insbesondere eine Konditioniermittelzubereitung, in einem zusätzlichen Container enthält. Diese Konditioniermittelzubereitung enthält vorteilhafterweise mindestens ein Konditioniermittel, ausgewählt aus der Gruppe von kationischen Polymeren, Siliconderivaten und Ölen. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, welche das Abteilen von Faserbündeln bzw. Strähnchen aus der Gesamtmenge der Fasern erlaubt und vereinfacht.

Bezüglich des erfindungsgemäßen kosmetischen Mittels in dem Container C1 und der Oxidationsmittelzubereitung in dem Container C2 gilt mutatis mutandis das zu den erfindungsgemäßen kosmetischen Mitteln Gesagte.

Ein weiterer Gegenstand der vorliegenden Erfindung ist ein Verfahren zum Färben von keratinischen Fasern, wobei das Verfahren die folgenden Verfahrensschritte umfasst:

a) Bereitstellen eines erfindungsgemäßen kosmetischen Mittels (M1),
b) Bereitstellen einer Oxidationsmittelzubereitung (M2), enthaltend in einem kosmetisch verträglichen Träger mindestens ein Oxidationsmittel sowie mindestens eine Säure,
c) Vermischen des kosmetischen Mittels (M1) mit der Oxidationsmittelzubereitung (M2),
d) Applizieren der in Schritt c) erhaltenen Mischung auf die keratinischen Fasern und Belassen dieser Mischung für eine Zeit von 10 bis 60 Minuten, bevorzugt von 20 bis 45 Minuten, bei Raumtemperatur und/oder bei mindestens 30 °C auf den keratinischen Fasern,
e) Spülen der keratinischen Fasern mit Wasser und/oder einer reinigenden Zusammensetzung für 1 bis 5 Minuten, und
f) gegebenenfalls Applizieren eines Nachbehandlungsmittels auf die keratinischen Fasern und Ausspülen nach einer Zeit von 1 bis 10 Minuten.

Das erfindungsgemäße Verfahren zur Färbung keratinischer Fasern unter Verwendung mindestens zweier spezieller nichtionischer linearer Siliconpolymere resultiert in einer verbesserten Buntheit und/oder Farbtiefe von Farbnuancen gegenüber Verfahren, in welchen keine speziellen nichtionischen linearen Siliconpolymere eingesetzt werden. Hierdurch kann mit einer geringeren Menge an Farbstoffen die gleiche Buntheit erreicht werden, wie mit Verfahren des Standes der Technik, in welchen keine derartigen Siliconpolymere eingesetzt werden.

Unter Raumtemperatur ist dabei im Rahmen der vorliegenden Erfindung die Umgebungstemperatur zu verstehen, welche ohne Einwirkung von externer Wärme vorherrscht und bevorzugt von 10 bis 39 °C beträgt. Die Wirkung der Färbe- und/oder Aufhellzubereitung kann durch externe Wärmezufuhr, beispielsweise mittels einer Wärmehaube, verstärkt werden. Die bevorzugte Einwirkdauer der Färbe- und/oder Aufhellzubereitung auf die keratinische Faser beträgt 10 bis 60 min, bevorzugt 20 bis 45 min. Nach Beendigung der Einwirkdauer wird das verbliebene Färbemittel aus den keratinischen Fasern mit Hilfe einer Reinigungszubereitung, welche bevorzugt mindestens ein kationisches und/oder anionisches und/oder nichtionisches Tensid enthält, und/oder Wasser ausgewaschen. Gegebenenfalls wird der Vorgang mit einem weiteren Mittel wiederholt. Nach dem Auswaschen werden die keratinischen Fasern gegebenenfalls mit einem Nachbehandlungsmittel, beispielsweise einem Konditioniermittel, gespült und mit einem Handtuch oder einem Heißluftgebläse getrocknet. Die Auftragung der Färbezubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbemittel mit einer Applikationshilfe auf die keratinischen Fasern aufzutragen.

Bezüglich des erfindungsgemäßen kosmetischen Mittels M1, der Oxidationsmittelzubereitung M2 sowie weiterer bevorzugter Ausführungsformen des erfindungsgemäßen Verfahrens gilt mutatis mutandis das zu den erfindungsgemäßen kosmetischen Mitteln sowie zu der erfindungsgemäßen Verpackungseinheit Gesagte.

Darüber hinaus ist ein weiterer Gegenstand der vorliegenden Erfindung die Verwendung eines erfindungsgemäßen kosmetischen Mittels zur Erhöhung der Buntheit und/oder Farbtiefe von Farbnuancen. Der Einsatz mindestens zweier spezieller nichtionischer linearer Siliconpolymere resultiert in einer erhöhten Buntheit und/oder Farbtiefe von Farbnuancen. Hierdurch kann die Menge an Farbstoffen in den erfindungsgemäßen kosmetischen Mitteln gegenüber Mitteln des Standes der Technik ohne derartige Siliconpolymere gesenkt werden, um eine identische Buntheit und/oder Farbtiefe zu erreichen wie mit oxidativen Färbemitteln des Standes der Technik.

Bezüglich weiterer bevorzugter Ausführungsformen der erfindungsgemäßen Verwendung gilt mutatis mutandis das zu den erfindungsgemäßen kosmetischen Mitteln sowie zu der erfindungsgemäßen Verpackungseinheit Gesagte.

Schließlich ist ein weiterer Gegenstand der vorliegenden Erfindung die Verwendung einer erfindungsgemäßen

Verpackungseinheit zur Herstellung eines kosmetischen Mittels zur Farbveränderung keratinischer Fasern mit erhöhter Buntheit und/oder Farbtiefe von Farbnuancen. Wie bereits zuvor ausgeführt, resultiert der erfindungsgemäße Einsatz mindestens zweier spezieller nichtionischer linearer Siliconpolymere in einer erhöhten Buntheit und/oder Farbtiefe von Farbnuancen, welche eine Reduzierung der Menge an Farbstoffen erlaubt.

Bezüglich weiterer bevorzugter Ausführungsformen der erfindungsgemäßen Verwendung gilt mutatis mutandis das zu den erfindungsgemäßen kosmetischen Mitteln sowie zu der erfindungsgemäßen Verpackungseinheit Gesagte.

Die nachfolgenden Beispiele sollen bevorzugte Ausführungsformen der Erfindung erläutern.

Beispiele:

1. Rezepturen

Zusammensetzungen der eingesetzten kosmetischen Mittel (Öl-in-Wasser-Emulsionen, alle Mengen in Gew.-%). Das in den nachfolgenden Formulierungen verwendete nichtionische lineare Siliconpolymer ist bevorzugt eine Mischung aus einem Siliconpolymer (SP1) der Formel (II) mit x = 1.650 bis 8.000, $R^2$ und $R^7$ = Methyl und einem mittleren Molekulargewicht $M_w$ von 122.000 bis 628.000 Da sowie einem nichtionischen linearen Siliconpolymer (SP2) der Formel (III) mit y = 40 bis 1.300, $R^2$ und $R^7$ = Methyl und einem mittleren Molekulargewicht $M_w$ von 3.000 bis 97.000 Da.

| Rohstoff | V1 | E1* | E2* |
|---|---|---|---|
| Xanthan Gum | 0,05 | 0,05 | 0,05 |
| 2-Octyldodecanol | 2,3 | 2,3 | 2,3 |
| Lanette N [a)] | 14 | 14 | 14 |
| Cetearyl Alkohol | 3,9 | 3,9 | 3,9 |
| Glycerinmonostearat | 6,0 | 6,0 | 6,0 |
| Glycerol 99,5 % | 2,0 | 2,0 | 2,0 |
| Kokosamidopropylbeatin, 40%ig | 2,0 | 2,0 | 2,0 |
| Monoethanolamin | 4,5 | 4,5 | 4,5 |
| 2-Amino-2-methylpropanol | 0,1 | 0,1 | 0,1 |
| Natriumsulfit, wasserfrei | 0,2 | 0,2 | 0,2 |
| Caramelsirup, 75%ig | 0,1 | 0,1 | 0,1 |
| Traubenkernöl | 1,0 | 1,0 | 1,0 |
| p-Toluylendiaminsulfat | 0,03 | 0,03 | 0,03 |
| 4-Amino-3-methylphenol | 0,3 | 0,3 | 0,3 |
| 1,3-Benzoldiol | 0,04 | 0,04 | 0,04 |
| 1-Naphthol | 0,09 | 0,09 | 0,09 |
| 5-Amino-2-methylphenol | 0,2 | 0,2 | 0,2 |
| 2-Amino-6-chloro-4-nitrophenol | 0,2 | 0,2 | 0,2 |
| Nichtionisches lineares Siliconpolymer ** | - | 0,60 | 1,2 |
| Wasser, vollentsalzt | ad 100,00 | ad 100,00 | ad 100,00 |
| * erfindungsgemäß<br>** Aktivsubstanz<br>[a)] INCI-Bezeichnung: Cetearyl alcohol, Sodium cetearyl sulfate (BASF) | | | |

Die Fettbasis wurde jeweils zusammen bei 80°C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei der Rezeptur V1 handelt es sich um eine nicht erfindungsgemäße Vergleichsrezeptur ohne nichtionisches lineares Siliconpolymer. Die Rezepturen E1

und E2 sind erfindungsgemäße Beispiele.

Oxidationsmittelzubereitung O1 (alle Mengen in Gew.-%)

| Rohstoff | O1 |
|---|---|
| Dinatriumpyrophosphat | 0,10 |
| Dipicolinsäure | 0,10 |
| Kaliumhydroxid 50% | 0,22 |
| 1-Hydroxyethan-1,1-diphosphonsäure 60% | 0,25 |
| Emulgade F [b)] | 4,0 |
| Cetearyl Alcohol | 0,5 |
| Ceteareth-20 | 0,5 |
| Bienenwachs | 0,3 |
| Isopropylmyristat | 10 |
| Wasserstoffperoxid 50 % | 11 |
| Wasser vollentsalzt | ad 100 |
| [b)] INCI-Bezeichnung: Cetearyl alcohol, PEG-40 Castor oil, Sodium cetearyl sulfat (BASF) | |

2. Erhöhte Buntheit und Farbtiefe durch den Zusatz einer Mischung aus nichtionischen linearen Siliconpolymeren mit verschiedenen mittleren Molekulargewichten $M_w$

Zur Herstellung der oxidativen Färbemittel für die Bestimmung der Buntheit und Farbtiefe wurden die kosmetischen Mittel V1, E1 und E2 jeweils im Gewichtsverhältnis 1:1 mit der obigen Oxidationsmittelzubereitung O1 vermischt.

Die auf diese Weise hergestellten oxidativen Färbemittel wurden jeweils in definierter Menge (4 g oxidatives Färbemittel pro 1 g Yakhaar) auf Yakhaar-Strähnen aufgetragen (jeweils 12 Strähnen pro oxidativem Färbemittel) und verblieben für eine Einwirkdauer von 30 Minuten bei 32 °C auf den Haarsträhnen. Anschließend wurden die verbliebenen Mittel jeweils 2 Minuten lang mit lauwarmem Wasser aus den Haarsträhnen ausgespült, die Strähnen zunächst mit einem Handtuch getrocknet und anschließend trocken geföhnt.

Alle Strähnen wurden mit einem Farbmessgerät der Firma Datacolor, Typ Spectraflash 450, vermessen. Die für die Beurteilung der Buntheit und Farbtiefe herangezogenen Werte dC und dL ergeben sich aus den an der jeweiligen Strähne gemessenen L*a*b-Farbmesswerten wie folgt:

$$dC = [(a_i-a_0)^2+(b_i-b_0)^2]^{1/2} \text{ bzw. } dL = L_i-L_0$$

$a_0$, $b_0$ und $L_0$ sind hierbei jeweils die Mittelwerte der aus den 12 Messungen ermittelten Farbmesswerte der mit dem oxidativen Färbemittel V1 behandelten Yakhaar-Strähnen, während $a_i$, $b_i$ und $L_i$ jeweils die Mittelwerte der Farbmesswerte nach der oxidativen Färbung der Haarsträhnen mit den oxidativen Färbemitteln E1 und E2 darstellen.

Ein Indikator für die Buntheit der Ausfärbungen ist das Chroma. Je höher der dC-Wert ist, desto höher ist die Buntheit der Nuance. Die Buntheit kann optisch noch gesteigert werden, wenn sich die Farbtiefe ebenfalls erhöht, der dL-Wert also geringer wird. In nachfolgender Tabelle sind die dC-Werte sowie die dL-Werte für die Ausfärbungen unter Verwendung der kosmetischen Mittel V1, E1 und E2 dargestellt. Die Ausfärbungen mit den erfindungsgemäßen kosmetischen Mitteln E1 und E2, welche eine Mischung aus speziellen nichtionischen linearen Siliconpolymeren in einer Gesamtmenge von 0,6 Gew.-% bzw. 1,2 Gew.-% enthalten, weisen im Vergleich zu der Ausfärbung ohne spezielle nichtionische lineare Siliconpolymere (V1) eine verbesserte Buntheit und Farbtiefe auf.

| Oxidatives Färbemittel | dC | dL |
|---|---|---|
| E1 + O1 (1:1) | 1,41 | -3,27 |
| E2 + O1 (1:1) | 0,89 | -3,50 |

**Patentansprüche**

1. Kosmetisches Mittel zur Farbveränderung keratinischer Fasern, umfassend in einem kosmetisch verträglichen Träger

a) mindestens eine Verbindung, ausgewählt aus der Gruppe von Oxidationsfarbstoffvorprodukten, direktziehenden Farbstoffen sowie deren Mischungen,
b) mindestens ein nichtionisches lineares Siliconpolymer der Formel (I)

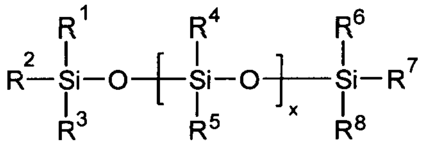
(I),

worin

x für ganze Zahlen von 10 bis 10.000 steht, und
$R^1$ bis $R^7$, jeweils unabhängig voneinander, für Wasserstoff, eine Hydroxygruppe, eine lineare oder verzweigte $C_{1-4}$-Alkylgruppe oder eine lineare oder verzweigte $C_{1-20}$-Hydroxyalkylgruppe stehen,

wobei das kosmetische Mittel mindestens zwei voneinander verschiedene lineare nichtionische Siliconpolymere der Formel I in einer Gesamtmenge von 0,05 bis 1,0 Gew.-%, bezogen auf das Gesamtgewicht des kosmetischen Mittels, in Form einer Emulsion enthält, wobei die Emulsion Teilchen mit einem mittleren Teilchendurchmesser D50 von 0,3 μm bis 30 μm aufweist,
a) wobei das erste lineare nichtionische Siliconpolymer (SP1) die Formel (II) sowie ein mittleres Molekulargewicht Mw von 122.000 bis 628.000 Da aufweist

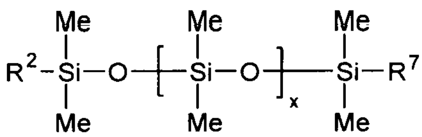
(II),

worin

$R^2$ und $R^7$, jeweils unabhängig voneinander, für eine Methylgruppe stehen, und
x für ganze Zahlen von 1.650 bis 8.500, steht, und

b) wobei das zweite lineare nichtionische Siliconpolymer (SP2) die Formel (III) sowie ein mittleres Molekulargewicht Mw von 3.000 bis 97.000 Da aufweist

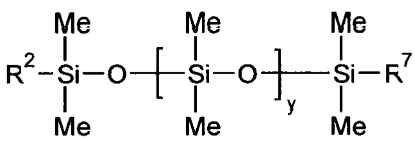
(III),

worin

$R^2$ und $R^7$, jeweils unabhängig voneinander, für eine Methylgruppe stehen, und
y für ganze Zahlen von 40 bis 1.300, steht.

2. Verpackungseinheit (Kit-of-Parts), umfassend - getrennt voneinander konfektioniert -

a) mindestens einen Container (C1), enthaltend ein kosmetisches Mittel nach Anspruch 1 und
b) mindestens einen Container (C2), enthaltend eine Oxidationsmittelzubereitung, welche in einem kosmetisch

verträglichen Träger mindestens ein Oxidationsmittel sowie mindestens eine Säure enthält.

**3.** Verfahren zum Färben von keratinischen Fasern, wobei das Verfahren die folgenden Verfahrensschritte umfasst:

a) Bereitstellen eines kosmetischen Mittels (M1) nach Anspruch 1,
b) Bereitstellen einer Oxidationsmittelzubereitung (M2), enthaltend in einem kosmetisch verträglichen Träger mindestens ein Oxidationsmittel sowie mindestens eine Säure,
c) Vermischen des kosmetischen Mittels (M1) mit der Oxidationsmittelzubereitung (M2),
d) Applizieren der in Schritt c) erhaltenen Mischung auf die keratinischen Fasern und Belassen dieser Mischung für eine Zeit von 10 bis 60 Minuten, bevorzugt von 20 bis 45 Minuten, bei Raumtemperatur und/oder bei mindestens 30 °C auf den keratinischen Fasern,
e) Spülen der keratinischen Fasern mit Wasser und/oder einer reinigenden Zusammensetzung für 1 bis 5 Minuten, und
f) gegebenenfalls Applizieren eines Nachbehandlungsmittels auf die keratinischen Fasern und Ausspülen nach einer Zeit von 1 bis 10 Minuten.

## Claims

**1.** A cosmetic agent for changing the color of keratin fibers, comprising, in a cosmetically acceptable carrier,

a) at least one compound selected from the group of oxidation dye precursors, substantive dyes and mixtures thereof,
b) at least one non-ionic linear silicone polymer of formula (I)

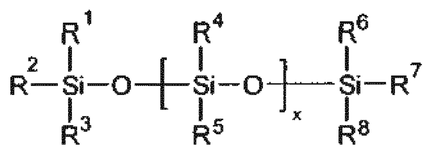
(I),

in which

x represents integers from 10 to 10,000, and
$R^1$ to $R^7$, each independently of one another, are hydrogen, a hydroxy group, a linear or branched $C_{1-4}$ alkyl group or a linear or branched $C_{1-20}$ hydroxyalkyl group, wherein the cosmetic agent comprises at least two mutually different linear non-ionic silicone polymers of formula I in a total amount of 0.05 to 1.0 wt.%, based on the total weight of the cosmetic agent, in the form of an emulsion, wherein the emulsion has particles having an average particle diameter D50 of 0.3 $\mu$m to 30 $\mu$m,

a) wherein the first linear non-ionic silicone polymer (SP1) has the formula (II) and an average molecular weight Mw of 122,000 to 628,000 Da,

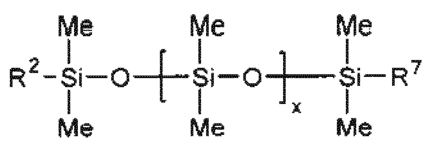
(II),

in which

$R^2$ and $R^7$, each independently of one another, represent a methyl group, and
x represents integers from 1,650 to 8,500, and

b) wherein the second linear non-ionic silicone polymer (SP2) has the formula (III) and an average molecular weight Mw of 3,000 to 97,000 Da,

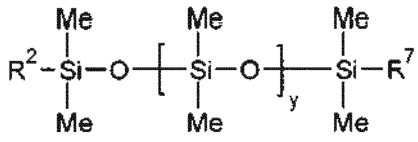

(III),

in which

$R^2$ and $R^7$, each independently of one another, represent a methyl group, and
y represents integers of from 40 to 1,300.

**2.** A packaging unit (kit-of-parts), comprising - assembled separately -

a) at least one container (C1) containing a cosmetic agent according to claim 1 and
b) at least one container (C2) containing an oxidizing agent preparation which contains, in a cosmetically acceptable carrier, at least one oxidizing agent and at least one acid.

**3.** A method for dyeing keratin fibers, wherein the method comprises the following method steps:

a) providing a cosmetic agent (M1) according to claim 1,
b) providing an oxidizing agent preparation (M2) containing, in a cosmetically acceptable carrier, at least one oxidizing agent and at least one acid,
c) mixing the cosmetic agent (M1) with the oxidizing agent preparation (M2),
d) applying the mixture obtained in step c) to the keratin fibers and leaving this mixture on the keratin fibers for a time of 10 to 60 minutes, preferably from 20 to 45 minutes, at room temperature and/or at at least 30 °C,
e) rinsing the keratin fibers with water and/or a cleaning composition for 1 to 5 minutes, and
f) optionally applying an aftertreatment agent to the keratin fibers and rinsing after a time of 1 to 10 minutes.

## Revendications

**1.** Composition cosmétique destinée à modifier la couleur des fibres kératiniques, dans un support cosmétique acceptable contenant

a) au moins une liaison choisie dans le groupe des produits précurseurs de colorants par oxydation, des colorants montant directement sur la fibre ainsi que leurs mélanges,
b) au moins un polymère de silicone linéaire non ionique de formule (I)

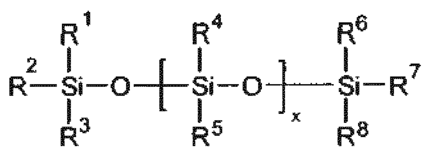

(I),

dans laquelle

x représente des nombres entiers compris entre 10 et 10 000, et
$R^1$ à $R^7$ représentent chacun indépendant l'un de l'autre, pour l'hydrogène, un groupe hydroxy, un groupe alkyle $C_{1-4}$ linéaire ou ramifié ou un groupe hydroxyalkyle en $C_{1-20}$ linéaire ou ramifié, ladite composition cosmétique comprenant au moins deux polymères de silicone non ioniques linéaires de formule I mutuellement différents en une quantité totale de 0,05 à 1,0 % en poids, par rapport au poids total de la composition cosmétique, sous forme d'émulsion, ladite émulsion comprenant des particules ayant un diamètre moyen D50 des particules de 0,3 μm à 30 μm,

a) dans laquelle le premier polymère de silicone linéaire non ionique (SP1) ayant la formule (II) et un poids moléculaire moyen Mw de 122 000 à 628 000 Da

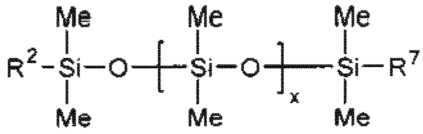

(II),

dans laquelle

$R^2$ und $R^7$, chacun indépendant l’un de l’autre, représentent un groupe méthyle, et
x est un nombre entier compris entre 1 650 et 8 500, et

b) où le deuxième polymère de silicone linéaire non ionique (SP2) ayant la formule (III) et un poids moléculaire moyen Mw de 3 000 à 97 000 Da

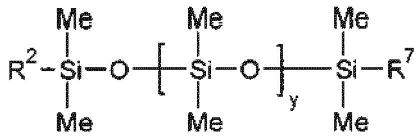

(III),

dans lequel

$R^2$ et $R^7$, chacun indépendant l’un de l’autre, représentent un groupe méthyle, et
y représente des nombres entiers compris entre 40 et 1 300.

**2.** Unité de conditionnement (Kits-of-Parts) comprenant, fabriqués séparément les uns des autres,

a) au moins un contenant (C1), comprenant une composition cosmétique selon la revendication 1 et
b) au moins un contenant (C2), comprenant une préparation d’agent oxydant qui comprend au minimum, dans un support cosmétique acceptable, un agent oxydant ainsi qu’un acide.

**3.** Procédé de coloration de fibres kératiniques, le procédé comprenant les étapes de procédé suivantes :

a) Fourniture d’une composition cosmétique (M1) selon la revendication 1,
b) Fourniture d’une préparation d’agent oxydant (M2) comprenant, dans un support cosmétique acceptable, au moins un agent oxydant et au moins un acide,
c) Mélange de la composition cosmétique (M1) avec la préparation d’agent oxydant (M2),
d) Application du mélange obtenu à l’étape c) sur les fibres kératiniques et le fait de poser ce mélange de 10 à 60 minutes, de préférence de 20 à 45 minutes, à température ambiante et/ou au moins 30°C sur les fibres kératiniques,
e) Rinçage des fibres kératiniques avec de l’eau et/ou une composition détergente de 1 à 5 minutes, et
f) application d’un agent de post-traitement sur les fibres kératiniques et rinçage après une période de 1 à 10 minutes le cas échéant.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2006123564 A1
- US 2010037404 A1


### In der Beschreibung aufgeführte Nicht-Patentliteratur


- **LIU X. M.** Comparative Studies of Poly(DimethylSiloxanes) Using Automated GPC-MALDI-TOF MS and On-Line GPC-ESI-TOF MS. *Am. Soc. Mass. Spectrom.,* 2003, vol. 14, 195-202